# EUROPEAN PATENT SPECIFICATION

(11) **EP 0 765 662 B1**
(45) Date of publication and mention of the grant of the patent: **04.12.2002**
(21) Application number: 95921986.6
(22) Date of filing: 19.06.1995
(51) Int. Cl.: A61K 31/375, C07D 407/04, A61P 31/18

(54) **USE OF 5,6-O-BENZYLIDENE-L-ASCORBIC ACID OR SALTS THEREOF FOR THE MANUFACTURE OF A MEDICAMENT FOR THE TREATMENT OF HIV**
VERWENDUNG VON 5,6-O-BENZYLIDEN-L-ASCORBINSÄURE ODER DEREN SALZE ZUR HERSTELLUNG EINES MEDIKAMENTS ZUR BEHANDLUNG VON HIV
UTILISATION DE L'ACIDE 5,6-O-BENZYLIDENE-L-ASCORBIQUE OU SES SELS POUR LA FABRICATION D'UN MEDICAMENT POUR LE TRAITEMENT DU VIH

(30) Priority: 20.06.1994 JP 13744394
(43) Date of publication of application: 02.04.1997
(73) Proprietor: Kochi, Mutsuyuki, Matsudo-shi Chiba 271 (JP)
(72) Inventor: KOCHI, Mutsuyuki, Chiba 271 (JP); UENO, Yoshio, Tokyo 175 (JP); TANUMA, Seiichi, Tokyo 192-03 (JP)
(74) Representative: Tanty, François
(86) International application number: JP9501214
(87) International publication number: WO95035106

(56) References cited:
- EP-A- 0 344 880
- WO-A-95/28925
- JP-A- 1 313 476
- JP-A- 5 004 985
- JP-A- 6 040 989
- JP-A- 60 139 619
- DATABASE MEDLINE [Online] US NATIONAL LIBRARY OF MEDICINE (NLM), BETHESDA, MD, US AOKI K ET AL: "Sodium benzylideneascorbate induces apoptosis in HIV -replicatin U1 cells." retrieved from STN Database accession no. 94357260 XP002121015 & FEBS LETTERS, (1994 AUG 29) 351 (1) 105-8. ,

## Description

### TECHNICAL FIELD

This invention relates to the use of 5,6-O-benzylidene-L-ascorbic acid or a pharmaceutically acceptable salt thereof as active ingredient for the manufacture of a medicament for inhibiting the proliferation of acquired human immunodeficiency syndrome virus (hereinafter sometimes abbreviated as HIV), namely AIDS virus, and for inhibiting the replication of viral particles of HIV within human T-cells which have been infected with HIV.

### BACKGROUND ART

Acquired human immunodeficiency syndrome, namely AIDS, is a disease which is caused due to human T-cells being infected with AIDS virus, that is, HIV. A variety of agents for inactivating HIV (hereinafter simply referred to as anti-HIV agent) have been reported as prophylactic or remedial agent for AIDS. For example, AZT (azidothymidine), DDI (2',3'-dideoxyinosine), and IFN (interferon), as well as alkylating agent or antimetabolic agent which is effective as an anticancer agent (see Japanese Patent Application "KOKAI" Hei-1-38031), etc. are already known as the anti-HIV agent.

Since, however, any of these known anti-HIV agents is not satisfactory as a remedial agent for AIDS, there exists still a keen demand to provide such a new anti-HIV agent which has a low toxicity and a high HIV-inactivating activity.

### DISCLOSURE OF THE INVENTION

We, the present inventors, have energetically made our investigations with taking the above-mentioned circumstances into consideration. As a result, we have now found that 5,6-O-benzylidene-L-ascorbic acid (hereinafter sometimes referred to simply as benzylidene-ascorbic acid) or a pharmaceutically acceptable metal salt thereof is of a low toxicity and exhibits a high HIV-inactivating activity. On the basis of these findings, we have accomplished this invention which is disclosed in claim 1.

The invention is useful for therapeutic treatments of AIDS. 5,6-O-benzylidene-L-ascorbic acid or a salt thereof to be used as the active ingredient in the composition of this invention is a compound known in the art. One method for the preparation of 5,6-O-benzylidene-L-ascorbic acid is described in the "Steroids", Vol. 12, p. 309, (1968), and another method for the preparation thereof is disclosed in EP-A-0148094 and US patent No. 5,036,103 specification. Further, EP-A-0148094 and US patent No. 5,036,103 specification also teach that5,6-O-benzylidene-L-ascorbic acid or sodium, potassium and calcium salts thereof have an anticancer activity, with disclosing a process for the preparation of them.

Furthermore pharmaceutical compositions comprising 5,6-O-benzylidene-L-ascorbic acid or a pharmaceutically acceptable alkali metal or alkaline earth metal salt are known from EP-A-0344880.

### BEST MODE FOR CARRYING OUT THE INVENTION

As concrete examples of the salts of benzylidene-ascorbic acid useful as the active ingredient in the HIV-proliferation inhibiting composition according to this invention, there may be mentioned mono-sodium salt, di-sodium salt, mono-potassium salt, di-potassium salt and calcium salt. Particularly preferred is 5,6-O-benzylidene-L-ascorbic acid mono-sodium salt (abbreviated as SBA). SBA is a compound represented by the following formula (I) 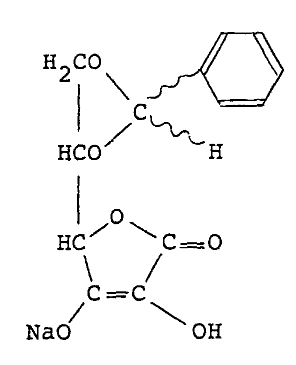 SBA as synthesized by a usual method exists in the form of a racemic mixture of two steric isomers, namely, (S)-SBA and (R)-SBA, and contains the (S)-form and the (R)-form in the mixing ratio of 2:1.

Benzylidene-ascorbic acid or a pharmaceutically acceptable salt thereof to be used as the active ingredient in this invention is of a low toxicity and exhibits a useful anti-HIV activity, as demonstrated in the Test Examples hereinafter given.

The invention is useful for the prophylactic treatments of human immunocompetent cells against their infections with HIV virus and also for the prophylactic or therapeutic treatments of acquired human immunodeficiency syndrome (AIDS). In cases where the anti-HIV composition according to this invention is to be used as a medicine for the above-mentioned purposes, the active ingredient compound may be formulated into a variety of pharmaceutical compositions in the form of powders, granules, tablets, capsules, syrups, injections, external remedies, suppositories, and the like, by incorporating the active compound appropriately with a pharmaceutically acceptable additive or additives such as pharmaceutically required additives, including carriers, activating agents, diluents, and others. These compositions may be administered either orally or parenterally to animals or human beings.

In the above-mentioned formulations of said anti-HIV compositions, the active ingredient compound to be used in this invention may be incorporated in an amount effective to achieve the purpose so intended. The dosage of the active ingredient to be administered may vary depending upon the route of administration, symptom of disease, body weight, sex and age of patient to whom the active ingredient is administered. For instance, the dosage will be about 100-3000 mg per day for adult patient. However, the dosage so determined may be given continuously or intermittently so far as the concentration of the active compound in the blood does never exceed a certain determined value as prescribed in consideration of various conditions, including the results of animal tests, etc. The optimal dosage to be given and the number of administrations under particular conditions have to be determined by an expert physician in charge, in view of the above-mentioned guideline. The content of the active ingredient compound in the anti-HIV composition of this invention also may vary diversely depending upon the types of the formulations. Usually, the content of the active ingredient may be in the range of 0.1-99% by weight, preferably of 1-90% by weight. For example, with injection preparations, they may preferably contain the active ingredient compound usually in the range of 0.1-5% by weight. In cases of orally administrable preparations, the active ingredient compound may be formulated in the form of tablets, capsules, powders, granules, dry syrups, liquid preparations, syrups, etc. by associating the active ingredient compound with the above-mentioned solid carrier(s) or liquid carrier(s). With the capsules, tablets, granules and powders, the content of the active ingredient compound may usually be 3-99% by weight, preferably 5-90% by weight, the balance being the carrier(s) incorporated. Preferably, the anti-HIV composition according to this invention contains as the active ingredient 5,6-O-benzylidene-L-ascorbic acid mono-sodium salt in an amount of 0.1-99% by weight based on the weight of the composition.

Excipients and carriers as incorporated into the anti-HIV composition according to this invention may be selected from those pharmaceutically acceptable ones, and the nature and composition of them may be determined depending upon the route and method of administration. For instance, as liquid carriers, there may be used water, alcohols or animal or vegetable oils such as soybean oil, sesame oil, mineral oils, or synthetic oils and the like. As solid carriers, there may be used saccharide such as maltose, sucrose, etc., amino acids such as lysine, etc., cellulose derivatives such as hydroxypropyl cellulose, etc., polysaccharide such as cyclodextrin, etc., organic acid salts such as magnesium stearate, and others.

In cases where the HIV-inactivating composition of this invention is formulated as injection preparations, it is usually desirable that liquid carriers are physiological saline, a variety of buffer solutions, solutions of saccharide such as glucose, inositol, mannitol, etc., glycols such as ethylene glycol, polyethylene glycol, and the like. Such injection preparations also can be formulated in the form of a lyophilized preparation in association with saccharide such as inositol, mannitol, glucose, mannose, maltose, sucrose, etc., and amino acids such as phenylalanine. The lyophilized preparation may be applied by dissolving the said preparation in a solvent suitable for injection purposes, such as sterilized water, physiological saline, glucose solution, electrolyte solution, amino acid solution, etc., which are applicable as a liquid for intravenous injection administrations.

According to a second aspect of this invention, there is provided a method for inhibiting the proliferation of HIV in human T-cells as infected with HIV, which comprises treating such human T-cells as infected with HIV, with 5,6-O-benzylidene-L-ascorbic acid mono-sodium salt in an effective amount to inhibit the replication of the viral particles of HIV in the human T-cells as infected with HIV.

In cases where there occurs a possibility or risk that a volume of blood which has been taken out from human body and stored outside the body would contain the T-cells as infected with HIV, the aforesaid method for inhibiting the proliferation of HIV according to the second aspect of this invention is utilizable, when said method is applied to the stored blood, in such a manner that the viral particles of HIV within the T-cells as infected with HIV which might exist in the said stored blood can be inhibited from being replicated, in other words, the proliferation of HIV can be inhibited from taking place.

5,6-O-Benzylidene-L-ascorbic acid mono-sodium salt (i.e. SBA) can serve effectively for therapy of a variety of cancers, if it is repeatedly administered to patients bearing a cancer by intravenous or intraperitoneal injection, as described in US patent No. 5,036,103 specification. As is appreciated from this, SBA can maintain its activity stably in the blood stream, when it is introduced into a blood vessel.

In cases where the aforesaid method for inhibiting the proliferation of HIV according to the second aspect of this invention is conducted in such a manner that an effective amount of SBA is administered to an animal or human body as infected with HIV through intravenous injection or other administration route, it is expectable that the presence of an effective amount of SBA as administered to or transferred into the blood stream in vivo which would contain the T-cells infected with HIV will be able to inhibit the replication of the viral particles of HIV, namely the proliferation of HIV in the T-cells by SBA being brought into contact with the T-cells as infected with HIV.

The following Formulation Examples and Test Examples are given in order to illustrate further in detail this invention, which is not limited to these Examples.

| Formulation Example 1 Preparation of tablets | | |
|---|---|---|
| (1) | SBA (i.e. 5,6-O-benzylidene-L-ascorbic acid mono-sodium salt) | 50 g |
| (2) | Magnesium metasilicate aluminate | 14 g |
| (3) | Maize starch | 21 g |
| (4) | Lactose | 35 g |
| (5) | Crystalline cellulose | 60 g |
| (6) | Carboxymethyl cellulose (CMC) calcium salt | 18 g |

The above-mentioned ingredients were homogeneously mixed to give a mixture to which then magnesium stearate (2 g) was added, and the mixing was further continued. The resulting powdery mixture was pressed into tablets each weighing 200 mg. These tablets may be colored, if necessary, with a conventional film-coating agent which is soluble in stomach (for example, polyvinylacetal diethylaminoacetate) or with an edible colorant.

| Formulation Example 2 Preparation of capsules | | |
|---|---|---|
| (1) | SBA | 1000 g |
| (2) | Lactose | 960 g |
| (3) | Magnesium stearate | 40 g |

The above-mentioned ingredients were homogeneously mixed, and the resulting powdery mixture was packed into hard gelatine capsules so that each capsule contained 200 mg of the mixture.

| Formulation Example 3 Preparation of injections | | |
|---|---|---|
| (1) | SBA | 100 mg |
| (2) | Glucose | 100 mg |
| (3) | Physiological saline | |
| | Balance to give total | 10 ml |

SBA and glucose were dissolved in pysiological saline. The solution was filtered through a membrane filter and then again ultra-filtered for the expulsion of bacteria. The resulting filtrate was poured into each of vials under sterile conditions, after which the vials were filled with nitrogen gas and then sealed to afford vials containing injectable solutions for intravenous purposes.

### Test Example 1 Evaluation of toxicity

SBA in the form of a solution in a physiological saline (containing 100 µg of SBA/ml) was administered intravenously to ICR strain mice (5 week-aged, male, 5 mice in each group) at a dosage of 1000 mg per kg of the body weight of mice tested. None of the mice thus treated died. Thus, it has now been found from this animal test that 5,6-O-benzylidene-L-ascorbic acid or its sodium salt exhibits an extremely low toxicity.

### Test Example 2

As the cell to be tested here for estimation of the occurrence of HIV in this Example, there were used such U1 cells which had been prepared by introducing 2 copies of the gene DNA of HIV-1 (HTLV-IIIB strain) into U937 cells (human monocytic blast-like cells, namely one sort of human T-cells). The U1 cell is an HIV potentially-infected T-cell wherein the HIV-1 gene is present merely as provirus. The U1 cell is such a type of the T-cell which does not bring about the replication of the viral particles of HIV, namely does not involve the occurrence or development of HIV, so long as the HIV-potentially infected U1 cell is not stimulated by a cell stimulator, such as TPA (phorbol ester) or TNF (tumor necrosis factor) and the like.

The above-mentioned U1 cells were seeded and incubated in Costar 48-well-plates under such different conditions as indicated below. Thus, the U1 cells were seeded in a culture medium and were incubated in the presence of 5,6-O-benzylidene-L-ascorbic acid mono-sodium salt (hereinafter abbreviated as SBA) as incorporated at different concentrations by addition of SBA to the culture medium, or the U1 cells were incubated in the absence of SBA. The incubation of these U1 cells was further effected with such both two types of those U1 cells, namely with such one type of U1 cells which had been stimulated further by addition of TPA at various concentrations thereto, and with such another type of U1 cells which had not been stimulated by TPA.

When the U1 cells as stimulated by the addition of TPA were incubated, replication of the viral particles of HIV took place in considerable numbers of the U1 cells, resulting in the production of the HIV antigen within the cells, so that said cells thus became such HIV-positive cells which could sprout and release the viral particles of HIV therefrom. This replication of the viral particles of HIV can be detected by an indirect fluorescent antibody technique as illustrated below.

The U1 cells were thus incubated under the different conditions as explained in the above, including such a case where both SBA and TPA were not added; such a case where SBA was added, but TPA was not added; such a case where SBA was not added, but TPA was added; and such as case where both SBA and TPA were added, respectively. Then, the replication of the viral particles of HIV within the U1 cells, and the release of the viral particles of HIV from the U1 cells so incubated under the different conditions as above mentioned were detected by means of the indirect fluorescence technique in which there were employed a mouse monoclonal antibody having an anti-HIV-1 activity as a primary antibody and also employed an FITC-rabbit-originated human IgG antibody as a secondary antibody. The number of the U1 cells which could be detected as the HIV-positive cells was calculated in term of percentage (%) on the basis of the total numbers of the U1 cells as subjected to the test, and it was expressed as the rate (%) of occurrence or appearance of the HIV-1 positive cells.

The test results so obtained are shown in Table 1.

**Table 1**

| Concentration of SBA in the culture medium (µg/ml) | Rate (%) of occurrence of HIV-1 positive cells | |
|---|---|---|
| | The case where TPA was not added | The case where TPA (1 ng/ml) was added |
| 0 | 3 | 27 |
| 25 | 3 | 26 |
| 50 | 2 | 21 |
| 100 | 2 | 16 |
| 200 | 3 | 14 |
| 400 | 3 | 12 |
| 800 | 4 | 8 |

As is clear from the results of Table 1, SBA as added at concentrations ranging from 50 to 800 µg/ml is observed to be effective to reduce the rate (%) of occurrence of the HIV-1 positive cells and to inhibit the replication of the viral particles of HIV, depending upon the concentration of SBA, in such experiments where the U1 cells were incubated in the presence of TPA added. In these experiments, the concentration of SBA required to achieve 50% inhibition of the replication of the HIV viral particles is at about 400 µg/ml.

In account of the results above, it has been confirmed that SBA, and, in general, 5,6-O-benzylidene-L-ascorbic acid or salts thereof, possess such an activity for inhibiting the replication of the viral particles of HIV within the host cells as potentially infected with HIV, and hence exhibits an anti-HIV activity.

### Test Example 3

It is known that stimulation of HIV gene by TPA and tumor necrosis factor (TNF) can activate the HIV gene which is latent as the HIV-provirus within the T-cells as infected with HIV and said stimulation can thus strongly induce the progresses of sprouting and release of the viral particles of HIV. It is considered that said activation of the HIV gene for the occurrence or developement of HIV virus would be caused by such an oxidation stress which is brought about within the host cells by any stimulation given from outside the host cells as above-mentioned. Further, such discoveries and observations have been reported that NFₖB, which is a nuclear factor wthin the cells, can play important part in the proliferation of HIV virus, and that the oxidation-reduction status of the NFₖB is important for the activation of this factor NFₖB, and so on. Then, examination as to whether or not SBA possesses an inhibitory activity against the proliferation of HIV was done by using the following test methods at the level of cultured cells.

### (a) Test of examining cytotoxicity of SBA against various kinds of T-cells

A3.01 cells (HAT-susceptible CEM cell strain), A3.01 cells as infected with HIV, and the above-mentioned U1 cells were cultivated, respectively, in an improved RPM1-1640 media containing SBA added at different concentrations. After the cultivation, the number of the cells under test which still survived was determined under a polarizing microscope by a trypan blue dye-exclusion method. The rate (%) of survival of the cells was then calculated and is shown in Table 2.

**Table 2**

| Concentration of SBA in the culture medium | Rate of survival of the cells (%) | | |
|---|---|---|---|
| (µg/ml) | U1 cells | A3.01 cells | A3.01 cells as infected with HIV |
| 0 | 80 | 92 | 100 |
| 100 | 90 | 97 | 98 |
| 500 | 100 | Not tested | 24 |
| 800 | 83 | Not tested | 3 |
| 1000 | 92 | 70 | Not tested |

In the above-mentioned tests of the cytotoxicity of SBA against the A3.01 cells, the HIV-infected A3.01 cells and the U1 cells, SBA exhibits no substantial cytotoxicity. The cytotoxicity of SBA against the A3.01 cells, at a high SBA concentration of 1000 µg of SBA/ml, was such that the rate of survival of said cells was about 70%. The above results of testing the cytotoxicity of SBA indicate that the cytotoxicity of SBA against the above various cells is fairly low. On the other hand, with the HIV-infected A3.01 cells, it is seen that the presence of SBA at concentrations in a range of from 500 to 800 µg of SBA/ml gives the rate of survival of the HIV-infected A3.01 cells at a level as low as 3-24%. This indicates that SBA exhibits an activity inhibitory to the viability of the HIV-infected A3.01 cells.

### (b) Tests of examining the inhibitory activities of SBA against infestion of HIV to mitogen-derived blast cells of human periphery blood monocytic cell (PBMC) and against proliferation of HIV in the HIV-infected blast cells of PMBC as well as against replication of the viral particles of HIV in the HIV-infected blast cells of PBMC, where HIV was made to infest the blast cells of PBMC of which the blastogenesis has been induced by a mitogen.

Such blast cells of PBMC, which were blasto-genetically transformed by activation with PHA (phorbol ester) as a mitogen, were cultivated and then made to be infested with HIV either by inoculation of HIV or by inoculation of HIV which had been pretreated with SBA. The HIV-infected cells so obtained continued further to be incubated in the culture media containing different concentrations of SBA. Then, the supernatant of the resulting cultures was separated, and the amount of the viral particles of HIV as released in the supernatant so obtained was measured. This measurement of the released amount of the viral particles of HIV was made by determining the amount of HIV-p24 antigen, i.e. a structural protein of HIV, existing in the supernatant, as a substitute index which can represent the amount of the viral particles of HIV so released in said supernatant. The test results are shown in Table 3.

**Table 3**

| Concentration of SBA in the culture medium | Amount of HIV-p24 antigen (pg/ml) | |
|---|---|---|
| (µg/ml) | Cultivation of the cells as infected with HIV | Cultivation of the cells as infected with the SBA-pretreated HIV |
| 0 | 1100 | 1200 |
| 10 | 1500 | 1240 |
| 50 | 1400 | 1520 |
| 100 | 380 | 1430 |

When the anti-HIV activity of SBA was estimated in terms of the amount of the HIV-p24 antigen as formed and appearing in the blast cells of the PHA-activated PBMC which were infested with HIV, as in the tests above-mentioned, it has been found that SBA exhibited an effective activity inhibitory to the production of the HIV antigen at the concentration of SBA of 100 µg/ml. Thus, the results of Table 3 show that it have been admitted that SBA can act effectively to inhibit the replication of the viral particles of HIV. Contrary to this, however, in the another test where the blast cells of PBMC cell as infected with the SBA-pretreated HIV was cultivated in the presence of SBA, no appreciable effect of SBA to inhibit the replication of the viral particles of HIV can be observed. In view of all these test results, it appears that the inhibitory activity of SBA against the replication of HIV-1 is not attributable to that SBA directly acts on HIV so as to make lose the infectivity (infectious power) of HIV.

### (c) Test of examining the inhibitory activity of SBA against such replication of the viral particles of HIV which is induced when a tumor necrosis factor (TNF-α) is introduced into the HIV-infected A3.01 cells

AIDS virus, HIV_{BRU} (its reverse transcriptase activity, 0.03 cpm/cell) and 6 x 10⁶ cells of the A3.01 cell (HAT-susceptible CEM cell strain) were mixed and reacted together in a culture medium at 37°C for 1 hour, and thereafter the mixed cells were washed with PBS to remove the excess of the particles of HIV virus therefrom. The A3.01 cells (5 x 10⁵ cells) which had been so infected with HIV were then cultivated in various culture media containing TNF-α and SBA at different concentrations of them. The amount of the HIV-p24 antigen (an HIV-structural protein of HIV) which was released in the culture supernatant from the cultivated cells under test was periodically measured during the period of between 0 day to 5th day of the cultivation.

The culture medium used for the above-mentioned cultivation of the A3.01 cells was an RPM1-1640 medium comprising 10% of heat-treated embryonic bovine serum, and minor amounts of penicillin, streptomycin, glutamine and mercaptoethanol.

The amount of the HIV-p24 antigen produced was measured with lapse of time, and the relationship of the amount of the HIV-p24 antigen to the concentration of SBA is summarily tabulated in Table 4 below.

**Table 4**

| Concentration of SBA in the culture medium (µg/ml) | Amount of the HIV p24 antigen (pg/ml) | | | | | |
|---|---|---|---|---|---|---|
| | The day of the measurement | | | | | |
| | 0th | 1st | 2nd | 3rd | 4th | 5th |
| 0 | 0 | 0 | 0 | 1750 | 3500 | 5300 |
| 1 | 0 | 0 | 0 | 1400 | 2600 | 4000 |
| 10 | 0 | 0 | 0 | 800 | 1500 | 2250 |
| 100 | 0 | 0 | 0 | 0 | 0 | 0 |
| AZT (2 µg/ml) | 0 | 0 | 0 | 0 | 0 | 0 |

In the tests above, the replication of the viral particles of HIV was examined in terms of the amount of the p24 antigen protein produced and appearing in the culture supernatant from the cultivated cells. Thus, the amount of the p24 antigen produced was measured periodically over 6 days after the A3.01 cells were infested with HIV. It was then observed that in the absence of SBA, the replication of the viral particles of HIV was induced in the A3.01 cells by stimulation with 10 units/ml of TNF-α added. In the presence of SBA, however, the replication of the viral particles of HIV was considerably inhibited at the 5th day from the HIV infection when SBA was present at such concentration as low as 1 µg/ml of SBA, and it is seen that the addition of 10 µg/ml of SBA could inhibit the replication of the HIV viral particles by 50%, in comparison with the cases when SBA was absent. The results of Table 4 further show that the addition of 100 µg/ml of SBA completely inhibited the replication of the viral particles of HIV.

Further, Table 4 shows also the result of such a comparative test wherein the cultivation of the HIV-infected A3.01 cells was effected in the presence of 2 µg/ml of AZT (a known anti-HIV agent which is utilized practically for the therapy of AIDS) in place of SBA. When comparison was made between SBA and AZT in respect of their effects inhibitory to the infection of the cells with HIV and to the replication of the viral particles of HIV, it is shown that SBA at the concentration of 100 µg/ml exhibited said effects to a same degree as AZT which was present at the concentration of 2 µg/ml. Thus, the effective amount of SBA has the activity to inhibit the replication of the viral particles of HIV within the cells to an extent as same as AZT, and from this it is expected that SBA is useful similarly to AZT for the therapeutic treatments of AIDS.

### (d) Tests for evaluating an inhibitory activity of SBA to the formation of giant cells from HIV-infected Molt-4 cells

In a similar manner to that in the above Test (c), Molt-4 cells (a CD4-positive human T-cell strain) were infected with AIDS virus, HIV_{BRU}. Thereafter, The HIV-infected Molt-4 cells were mixed with such Molt-4 cells which had no HIV-infection. And the mixture of these cells were cultivated at a high density of the mixed cells in the above-mentioned improved RPM1-1640 medium containing SBA at its different concentrations. Whereupon, there was examined the presence of cell-fused cell(s) (namely, the giant cells) which were formed by fusion of the HIV-infected cells with each other or by fusion of the HIV-infected cells with the HIV-non-infected cells. The formation and appearance of the giant cells was confirmed by microscopic observation. The size of such giant cells is clearly different from the size of the other usual cells. In the above-mentioned experiment system of mixed cultivation of the mixture of the HIV-infected Molt-4 cells with the HIV-non-infected Molt 4 cells in the presence of SBA added, it has been found that the precence of SBA at a concentration of SBA of 10 µg/ml or higher can inhibit the formation of the giant cells at a time of 20 hours of the cultivation.

From the results of Tests (b)-(d) above-mentioned, it is revealed that SBA so tested is a substance which possesses an inhibitory activity against the replication of the viral particles of HIV within the T-cells as infected with HIV. In the respective tests above, it has been demonstrated that SBA can also inhibit the formation of the giant cells in Molt-4 cells under the actions of HIV and the infestion of HIV to the mitogen-induced blastogenically transformed blast cells of PBMC, as well as the production of HIVp24_{gag} protein in the A3.01 cells, and so on. Namely,it is shown that SBA has an anti-HIV activity.

In particular, by the above-mentioned discoveries that SBA inhibits the formation of the giant cells in Molt-4 cells under the actions of HIV, and that such a very small amount as 1 µg/ml of SBA inhibits the proliferation of HIV by induction with TNF-α, it is suggested that SBA can inhibit an initial stage of the transcription of HIV-1 gene within such cells as infected with HIV, so that the developement of the virus-envelope protein of HIV is inhibited. In the initial stage of the transcription of HIV-1 gene, said NFₖB, i.e. the transcription regulating factor of the host cells, plays an important role. Therefore, there may be obtained a strong suggestion for the possibility that SBA has an activity inhibitory to the activation of NFₖB.

NFₖB is known to be a complex protein which is composed by the association of a molecule of protein having a molecular weight of about 50,000 (p50) with a molecule of protein having a molecular weight of about 65,000 (p65). It is also known that NFₖB is existing in cytoplasm of the cells in such status that the nucleic acid-bonding capability of the said complex protein, NFₖB, has been inactivated by combination of NFₖB with IₖB protein (with a molecular weight of 37,000) which is a nucleic acid-bonding capability-inhibiting factor. If the cells are subjected to a stimulation from outside thereof by the oxidation stress factor or an inflammatory cytokine such as TNF-α, etc., activated oxygen can be produced in the cells. The resulting appearance of the activated oxygen in the cells can render the cells to induce the occurrence of an enzyme capable of catching the activated oxygen as a protective reaction in vivo against said activated oxygen formed. Among such enzymes, there are those enzymes having an action to activate the nucleic acid-bonding capability of NFₖB. The NFₖB thus activated is then bonded to the transcription regulating site of the messenger RNA of the HIV gene which was existing as provirus, so that the activated NFₖB will increase the transcription of HIV to mRNA, resulting in the accelerated proliferation of HIV. SBA which has a strong anti-oxidizing capability is believed to behave under the above-mentioned situation, in such manner that SBA can catch and inactivate the activated oxygen in the cells, so that the occurrence of the activated oxygen-catching enzyme derived from the host cells will not be induced, with such consequences that the induction of the acitivation of the nucleic acid-bonding capability of NFₖB will then fail, and that eventually, SBA can inhibit the replication of the viral particles of HIV-1.

### INDUSTRIAL UTILIZABILITY

As described hereinbefore, it has now been found according to this invention that 5,6-O-benzylidene-L-ascorbic acid or a pharmaceutically acceptable salt thereof, particularly mono-sodium salt, exhibits an activity inhibitory to the replication of the viral particles of HIV in human T-cells as infected with HIV, and that broadly speaking, the aforesaid compounds possess an inhibitory activity against the proliferation of HIV. 5,6-O-Benzylidene-L-ascorbic acid or a salt thereof is effective and useful to treat such human T-cell-infecting AIDS virus so as to inhibit the AIDS virus, and is of a low toxicity and therefore can be expected to be useful for therapeutic treatments of AIDS.

## Claims

1. Use of 5,6-0-benzylidene-L-ascorbic acid or a pharmaceutically acceptable alkali metal salt or alkaline earth metal salt thereof, for the manufacture of a medicament for inhibiting the proliferation of HIV for the prophylactic treatments of human immunocompetent cells against their infections with HIV virus and also for the prophylactic or therapeutic treatments of acquired human immunodeficiency syndrome (AIDS).

## Patentansprüche

1. Verwendung von 5,6-O-Benzyliden-L-ascorbinsäure oder einem pharmazeutisch annehmbaren Alkalimetallsalz oder Erdalkalimetallsalz derselben zur Herstellung eines Medikamentes zur Hemmung der Proliferation von HIV für die prophylaktischen Behandlungen von menschlichen immunkompetenten Zellen gegen deren Infektionen mit HIV-Virus und auch für die prophylaktischen oder therapeutischen Behandlungen von erworbenem menschlichen Immunmangel-Syndrom (AIDS).

## Revendications

1. Utilisation de l'acide 5,6-O-benzylidène-L-ascorbique ou d'un sel de métal alcalin ou d'un sel de métal alcalino-terreux de celui-ci pour la fabrication d'un médicament pour inhiber la prolifération du VIH pour les traitements prophylactiques de cellules immunocompétentes humaines contre leurs infections avec le virus VIH et aussi pour les traitements prophylactiques ou thérapeutiques du syndrome d'immunodéficience humaine acquise (S.I.D.A.).
